# EUROPEAN PATENT SPECIFICATION

(11) **EP 1 982 191 B1**
(45) Date of publication and mention of the grant of the patent: **24.07.2013**
(21) Application number: 07705345.2
(22) Date of filing: 19.01.2007
(51) Int. Cl.: C12Q 1/37, G01N 33/68

(54) **MAST CELL CARBOXYPEPTIDASE AS A MARKER FOR ANAPHYLAXIS AND MASTOCYTOSIS**
MASTZELLEN-CARBOXYPEPTIDASE ALS MARKER FÜR ANAPHYLAXIE UND MASTOZYTOSE
UTILISATION DE LA CARBOXYPEPTIDASE MASTOCYTAIRE COMME MARQUEUR DE L'ANAPHYLAXIE ET DE LA MASTOCYTOSE

(30) Priority: 19.01.2006 GB 0601058
(43) Date of publication of application: 22.10.2008
(73) Proprietor: University of Southampton, Southampton, Hampshire SO17 IBJ (GB)
(72) Inventor: WALLS, Andrew, Finlay, Southampton, Hampshire SO15 5QJ (GB); ZHOU, Xiaoying, Southampton, Hampshire SO16 6HW (GB)
(74) Representative: Lord, Hilton David
(86) International application number: PCT/GB2007/050031
(87) International publication number: WO 2007/083166

(56) References cited:
- WO-A-2005/022157
- ZHOU X ET AL: "Mast cell carboxypeptidase as a new clinical marker for anaphylaxis" JOURNAL OF ALLERGY AND CLINICAL IMMUNOLOGY, vol. 117, no. 2, Suppl. S, February 2006 (2006-02), page S85, XP005359734 62ND ANNUAL MEETING OF THE AMERICAN-ACADEMY-OF-ALLERGY-ASTHMA-AND-IMM UNOLOGY; MIAMI BEACH, FL, USA; MARCH 03 -07, 2006 ISSN: 0091-6749

## Description

The present invention relates to use of mast cell carboxypeptidase as a marker for mast cell activation, more particularly, for example, as a blood sample marker for mast cell activation resulting in anaphylaxis and mastocytosis, which is not detectable by raised serum tryptase. Detection of elevated mast cell carboxypeptidase in blood samples is also proposed for reducing false negatives in relation to diagnosis of mastocytosis by picking up serum tryptase-negative cases.

### Background to the invention

In allergic reactions mast cells are triggered to release a range of agents including enzymes such as tryptase, chymase and carboxypeptidase. Allergic reactions frequently affect just localised areas of tissue (e.g. the lower airways in asthma, the skin in urticaria), but mast cell activation as a result of allergen exposure may be triggered on occasions in multiple organ systems (anaphylaxis) and such allergic reactions can be life-threatening within a very short period. Anaphylaxis may be triggered following ingestion of foods (e.g. peanuts), following insect stings (especially by bees and wasps) and following administration of medicines and anaesthetics. The potential for an individual who has suffered an anaphylactic reaction to suffer a further life-threatening allergic reaction on re-exposure to the relevant allergen means that it is important that anaphylaxis is correctly diagnosed. Moreover, the unnecessary fear of a repeated anaphylactic reaction can lead to unwanted changes in lifestyle or, where medication is implicated as a causal agent, to withholding of the treatment of choice.

In recent years, immunoassays for mast cell tryptase in serum samples have been helpful in diagnosing both anaphylaxis and mastocytosis, for example using the ImmunoCAP™ tryptase test as marketed by Pharmacia Diagnostics AB. Circulating levels of tryptase are rarely elevated except following anaphylactic shock or mastocytosis. However, it has become increasingly clear that elevation of serum tryptase concentration is not detectable in all cases of anaphylaxis using conventional detection protocols, particularly where food or certain medications are implicated as the causative agent.

The reason why ingested allergen induced anaphylaxis should commonly be associated with a poor or undetectable increase in serum tryptase is not clear. Experimental induction of anaphylaxis to peanuts has been shown to be associated with sharp elevations in tryptase levels in saliva and hence measurement of tryptase in saliva samples has been proposed as a means of diagnosing food-induced anaphylactic reactions (Buckley et al. (2000), Increased levels of tryptase in saliva following provocation of food-induced allergic reactions, J. Allergy Clin. Immunol. 105, S145).

Unlike tryptase which is present in all human mast cells, chymase and mast cell carboxypeptidase are restricted to a subpopulation of mast cells which predominates in the submucosal tissues of the lung and gut and in the skin. Thus, while both chymase and mast cell carboxypeptidase have previously been considered as markers for mast cell activation, detection of mast cell carboxypeptidase has previously only been suggested in conjunction with serum tryptase measurement, for example, as a means of providing evidence of differential activation of mast cell populations (Coombs et al. (2000), 'Sudden infant death syndrome: Could a healthy infant succumb to inhalation-anaphylaxis during sleep leading to cot death?', Cambridge Publications).

Published International Patent Application WO 2005/022157 concerns *in vitro* evaluation of vaccines, including assessment of potential for inducing anaphylaxsis by assessing markers of mast cell activation, including tryptase and mast cell carboxypeptidase, in blood samples exposed to the vaccine.

Mast cell carboxypeptidase was first purified from skin and stomach tissues and characterised as a monomeric metalloprotease with a molecular weight of about 34.5 kDa (Goldstein et al. (1989), Human mast cell carboxypeptidase - purification and characterization, J. Clin . Invest. 83, 1630-1636; Bunnett et al. (1992), Isolation of a neuropeptide-degrading carboxypeptidase from the human stomach, Gastroenterol. 102, 76-87). A molecular weight of 36.1 kDa and a net positive charge of 16 has been calculated from the cDNA-derived amino acid sequence of the catalytic portion (Reynolds et al. (1989), Cloning of cDNAs that encode human mast cell carboxypeptidase-A and comparison of the protein with mouse mast cell carboxypeptidase-A and rat pancreatic carboxypeptidases, Proc. Natl. Acad. Sci. USA 86, 9480-9484). Mast cell carboxypeptidase is a distinct form of carboxypeptidase but it has substrate specificity similar to that of pancreatic carboxypeptidase A, removing carboxyl terminal residues from a range of regulatory peptides including angiotension I, Leu⁵- enkephalin, kinentensin, neuromedin N and neurotensin (Goldstein et al. (1989) ibid; Goldstein et al. (1991), Human mast cell proteases hydrolyse neurotensin, kinetensin and leu5-enkephalin, Peptides 12, 995-1000; Bunnett et al. (1992) *ibid*). It is co-released from human mast cells with tryptase, chymase and cathepsin G and has been shown to be bound to proteoglycans in the same macromolecular complex as chymase (Goldstein et al. (1992), Protease composition of exocytosed human skin mast cell protease-proteoglycan complexes - tryptase resides in a complex distinct from chymase and carboxypeptidase, J. Immunol. 148, 2475-2482).

The inventors have prepared monoclonal antibodies specific for mast cell carboxypeptidase and have used these to detect mast cell carboxypeptidase in human serum samples indicative of both anaphylactic reactions and mastocytosis. In an initial investigation, out of 110 cases of suspected anaphylaxis that were serum tryptase-negative, elevated serum mast cell carboxypeptidase was found by a sandwich ELISA procedure in 70% of cases. Using the same immunoassay protocol, mast cell carboxypeptidase was also found to be a favourable serum marker for different forms and categories of mastocytosis and to detect cases that were serum tryptase-negative.

### Summary of the invention

In one embodiment, the present invention thus provides use of an immunoassay for detection of mast cell carboxypeptidase in a sample derived from an individual following suspected anaphylaxis, or having suspected mastocytosis, wherein said use is for diagnosis of anaphylaxis or mastocytosis and said sample is liable to contain mast cell carboxypeptidase released from mast cells as a result of anaphylaxis or mastocytosis but is a tryptase-negative, serum, plasma or saliva sample.

By "tryptase-negative" will be understood that tryptase released from mast cells is not detectable by conventional immunoassay, e.g. the ImmunoCAP™ tryptase test. The sample will be a serum or plasma sample, or it may also be a saliva sample.

The inventors have also for the first time established that mast cell carboxypeptidase may be elevated in saliva as a result of ingested allergen induced allergic reaction, e.g. a food-induced or oral medicament-induced anaphylaxis. Thus, there is also now provided a method of diagnosing an ingested allergen induced allergic reaction, including anaphylaxis, in an individual suspected of having suffered such a reaction which comprises determining by immunoassay mast cell carboxypeptidase present in a saliva sample from the individual, wherein determination of an elevated level of said carboxypeptidase in the simple is indicative of an allergic reaction.

### Detailed description

As indicated above, the inventive concept of using mast cell carboxypeptidase to reduce false negatives in diagnosis of conditions associated with release of mast cell products, more particularly anaphylaxis and mastocytosis, depended upon obtaining of antibodies specific for mast cell carboxypeptidase. Such monoclonal antibodies may be obtained in a conventional manner using purified mast cell carboxypeptidase as illustrated by the preparation and characterisation of such antibodies detailed in Example 1. An immunoassay employing such antibodies, or antigen-binding fragments thereof, for diagnostic purpose in accordance with the invention may take any known format A sandwich format may however be preferred, e.g. a sandwich ELISA format as utilised by the inventors and exemplified in Example 2.

Use of an immunoassay for mast cell carboxypeptidase to reduce the problem of false negative diagnosis of anaphylaxis arising from tryptase detection is not confined to particular allergens but it is envisaged will find most use in relation to suspected anaphylaxis arising from food allergens or allergic reactions to medications and anaesthetics or insect venom. Such an immunoassay may be applied to a serum, plasma or saliva sample. In applying the invention, it may be chosen to assay for mast cell carboxypeptidase after negative testing for tryptase by immunoassay in conventional manner in the same sample or an identical sample.

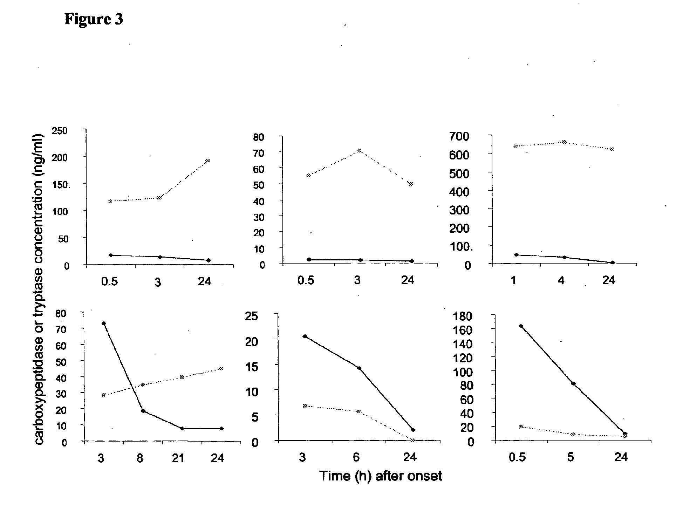

Timing of sample taking will desirably take account of the expected time course of appearance of mast cell carboxypeptidase and tryptase in blood or saliva following mast cell disintegration. Hence, it will generally be preferred to utilise samples obtained between about 30 minutes to 3 hours following onset of suspected anaphylaxis as recommended for conventional serum tryptase measurement for diagnosing anaphylaxis, although it may be found possible to use a sample obtained at a shorter time point, e.g. about 15 minutes. More desirably, at least a first sample may be obtained between about 30 minutes and 1 to 2 hours following onset of suspected anaphylaxis, although samples may be taken at longer time points for diagnostic purpose, e.g. up to 8 hours or longer, e.g. 24 hours. Desirably, after obtaining of a first sample further samples may be taken to confirm a decline in mast cell carboxypeptidase levels to baseline. It has previously been established that peak levels of tryptase in blood are not reached until 1 or 2 hours following allergen exposure (at least in the case of bee stings) with little if any rise being detectable prior to 30 minutes (Schwartz et al. (1989) Time course of appearance and disappearance of human mast cell tryptase in the circulation after anaphylaxis, J. Clin. Invest. 83, 5551-5555). Interestingly, however, it has now been found that whereas serum tryptase concentrations will almost invariably return to baseline levels by 24 hours, carboxypeptidase levels are frequently still elevated at this time point (see Figure 3). The measurement of serum carboxypeptidase levels could thus give an indication of anaphylaxis even if it has occurred about a day or more previously.

Although measurement of salivary tryptase has previously been recognised as a means for diagnosing food-induced anaphylaxis (Buckley et al. (2000), *ibid*.), no suggestion has previously been made to look for mast cell carboxypeptidase in saliva samples. As indicated above, it has now been established for the first time that mast cell carboxypeptidase can arise in saliva as a result of food-induced allergic reaction. Thus, there is now provided a method of diagnosing ingested allergen induced allergic reaction, especially ingested allergen induced anaphylaxis, in an individual suspected of having suffered such a reaction which comprises determining by immunoassay mast cell carboxypeptidase present in a saliva sample from the individual. The ingested allergen may be a food such as peanuts, shellfish or kiwi fruit. It may be a medicament. Samples of saliva will generally desirably be collected between about 15 minutes and 1 hour of ingestion of the suspected allergen.

As indicated above, mast cell carboxyypeptidase has also been found to be a favourable serum marker for various categories of mastocytosis and to detect cases that are serum tryptase-negative. Use of an immunoassay in accordance with the invention is envisaged as having particular utility, for example, in diagnosing any of the following categories of mastocytosis: diffuse cutaneous mastocytosis (DCM), urticaria pigmentosa (UP), aggressive systemic mastocytosis (ASM), mast cell leukaemia (MCL), indolent systemic mastocytosis (ISM) and mastocytosis without skin involvement associated to recurrent anaphylaxis or vascular collapse (SM-ANA).

The following examples illustrate the claimed embodiments with reference to the following figures:

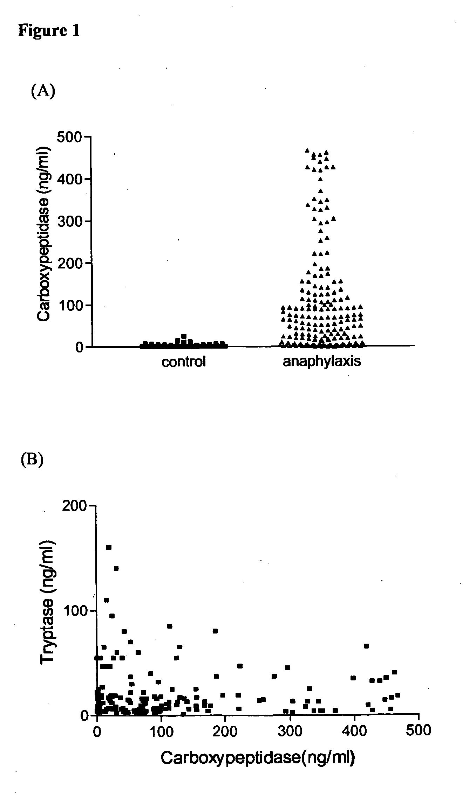

Figure 1: (A) Mast cell carboxypeptidase levels in serum samples taken from healthy controls and suspected anaphylactic patients within 8 hours of allergen exposure. (B) Comparison of carboxypeptidase and tryptase levels in the samples from patients with suspected anaphylaxis.
Figure 2: Levels of (A) carboxypeptidase and (B) tryptase in serum collected from patients with suspected anaphylaxis in response to injected drugs or anaesthetics, foods, wasp stings or in patients for whom the trigger could not be determined. The 95% upper percentile range is indicated for levels in healthy blood donors of carboxypeptidase (14 ng/ml) and of tryptase (13 ng/ml).
Figure 3: Levels of mast cell carboxypeptidase (---.---) and tryptase (-◆-) in serum collected from six individual cases at various periods following the onset of anaphylaxis.

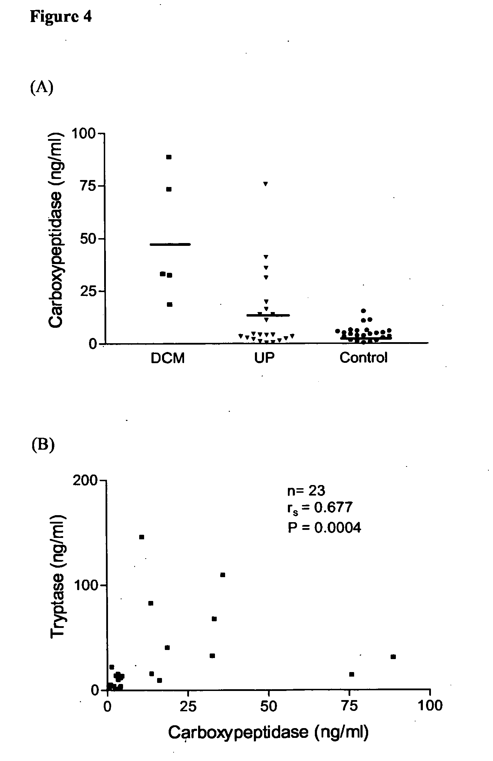

Figure 4: (A) Carboxypeptidase in serum from paediatric cases of diffuse cutaneous masctocytosis (DCM) and urticaria pigmentosa (UP) and from healthy children. (B) Comparison of carboxypeptidase and tryptase levels in samples from paediatric cases of mastocytosis.

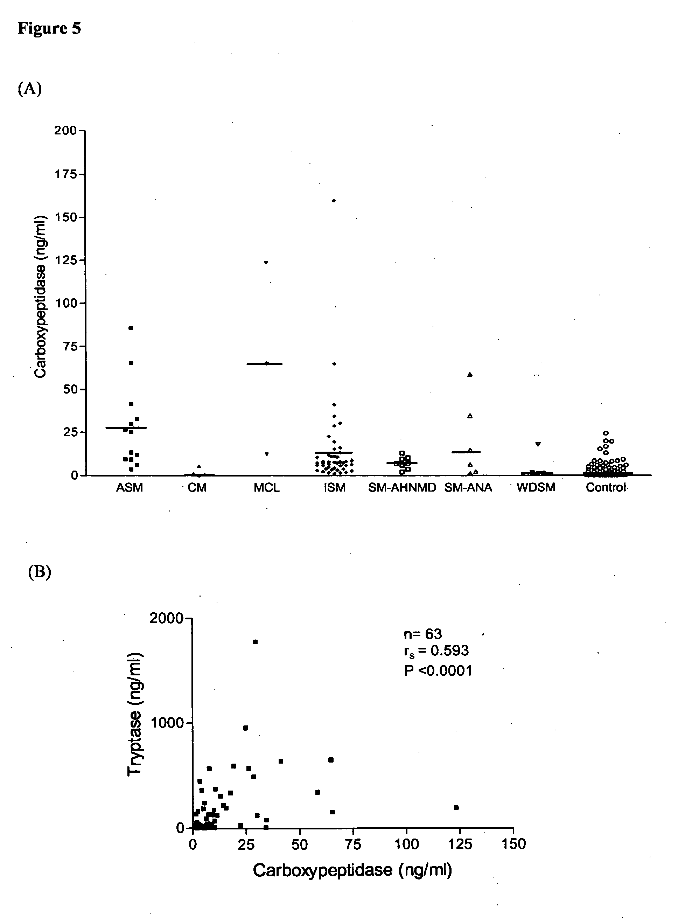

Figure 5: (A) Carboxypeptidase levels in serum from adults with aggressive systemic mastocytosis (ASM), cutaneous mastocytosis (CM), mast cell leukaemia (MCL), indolent system mastocytosis (ISM), systemic mastocytosis associated with haematological non-mast cell disease (SM-AHNMD), mastocytosis without skin involvement associated to recurrent anaphylaxis or vascular collapse (SM-ANA), well-differentiated systemic mastocytosis (WDSM), and from healthy control subjects. (B) Comparison of carboxypeptidase and tryptase levels in serum from adult patients with mastocytosis.

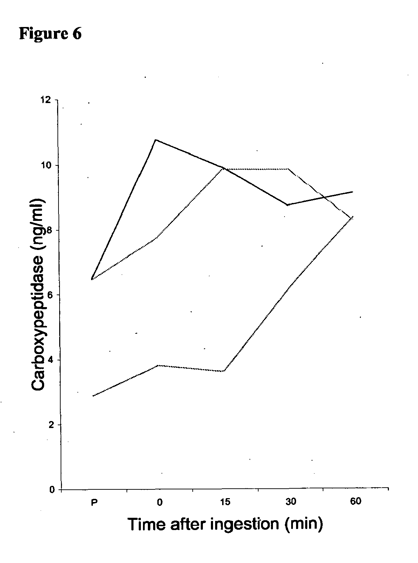

Figure 6: Levels of carboxypeptidase in saliva collected from three kiwi fruit allergic subjects either before (P) or following the placing of kiwi fruit into the mouth.

### Examples

### Example 1: Preparation and characterisation of antibodies specific for mast cell carboxypeptidase.

### Methods

### Purification of mast cell carboxypeptidase from skin extracts

Mast cell carboxypeptidase was purified from finely chopped extracts of human skin (obtained from macroscopically normal tissue at leg amputation) using a method adapted from that of Goldstein et al. (1989, *ibid).* Tissue extracts in a high salt buffer (2 M NaCl, 10 mM MES, pH 6.5) were applied to an affinity resin prepared by coupling potato tuber carboxypeptidase inhibitor (PCI) to cyanogen bromide activated agarose (Sigma, Poole, Dorset). After washing the column, carboxypeptidase was eluted with unconjugated PCI and dialysed to remove the inhibitor. The purity of the enzyme preparation was assessed by SDS-PAGE and silver staining (Pierce, Rockford, IL, USA). Proteolytic activity was determined by monitoring the cleavage of 0.5 M hippuryl-Phe-Arg (Sigma) at 260 nm.

### Cloning and preparation of recombinant carboxypeptidase

The carboxypeptidase gene was cloned from a human lung mast cell cDNA library and inserted into an over-expression vector. This was accomplished by PCR with the primers 5'-TTTGATGTTAAAGAATTCATCCCAGGCAGGCAC-3' (SEQ.ID NO. 1) and 5'-TTCCAAACAGAGGAAGCTTCTATAGGAAGTATG-3' (SEQ. ID NO. 2), insertion of amplimers into a pTrcHisA over-expression vector (Invitrogen, Paisley) and expression using *E. coli* Top 10 competent cells (Promega, Southampton). The carboxypeptidase cDNA was isolated and subcloned into a pET28a vector containing a His-Tag site (Merck Biosciences, Poole) using ECoRI and HindIII restriction enzymes and transformed into competent *E. coli* BL21 DE3 (Promega). The nucleotide sequence of the plasmid insert was investigated using the Big Dye Cycle Sequencing Protocol (Applied Biosystems, Foster City, CA, USA). Gene expression was induced using IPTG, and the recombinant protein was purified from bacterial lysates using a nickel ion affinity column (Qiagen, Crawley, West Sussex) eluting with imidazole. Bacterial lysates were subjected to SDS-PAGE and Western blotting using a monoclonal antibody to the His-Tag fusion partner and an enhanced chemiluminescence system (Pierce).

### Preparation of monoclonal antibodies

Splenocytes from a BALB/c mouse immunised with carboxypeptidase purified from human skin were fused with NS-1 myeloma cells and hybridoma cell supernatants were screened by ELISA as described previously (Buckley et al. (1999), The detection of mast cell subpopulations in formalin-fixed human tissues using a new monoclonal antibody specific for chymase, J. Pathol. 189, 138-143). Hybridoma cells producing antibodies reacting with human skin carboxypeptidase were subjected to three rounds of subcloning. Classes of the antibodies produced were determined using an isotyping kit (Sigma). Immunoglobulins were precipitated from cell supernatants using ammonium sulphate and purified by protein G-Sepharose (Sigma) chromatography as described previously (Buckley et al. (1999), *ibid).* For use in double labelling immunohistochemistry, purified antibody was conjugated to horseradish peroxidase (HRP) as described previously (Buckley et al. (1998), Mast cell subpopulations in the synovial tissue of patients with osteoarthritis: Selective increase in numbers of tryptase-positive, chymase-negative mast cells, J. Pathol. 186, 67-74).

### Immunoblotting

Bacterial lysates were blotted onto a PVDF membrane. The membrane was washed and blocked with 5% skimmed milk powder before incubation with 1 µg/ml carboxypeptidase-specific monoclonal antibody, or an isotype-marked control antibody (CC1 specific for chymase (Buckley et al. (1999), *ibid*). Binding of primary antibodies was detected using a peroxidase-conjugated rabbit antiserum to mouse immunoglobulins (Sigma) and an enhanced chemiluminesecnce substrate system (Pierce). Immunostaining was visualised by exposure of the blots to X-ray film (Kodak, Hemel Hempstead, Herts).

### Tissue processing and immunohistochemistry

Approval for these studies was granted by the Southampton and South West Hampshire Local Research Ethics Committees (241/01) and participants gave written informed consent. Endobronchial biopsy tissue was collected from volunteers with mild atopic asthma (n=6) and from healthy, non-atopic subjects (n=5). Tissues were embedded in glycol methacrylate (GMA) resin, and sequential 2µm sections were cut. Mast cells were identified using antibodies specific for tryptase (AA1; Walls AF et al. (1990) Immunohistochemical identification of mast cells in formaldehyde-fixed tissue using monoclonal antibodies specific for tryptase. J Pathology 162:119-126.), chymase (CC1) or the newly generated antibody to carboxypeptidase. Primary antibodies were detected by the sequential application of biotinylated polyclonal antibody to mouse immunoglobulins, streptavidin-horse radish peroxidase complexes and 9-aminoethyl carbazole (AEC).

Synovial tissue was obtained from the knees of patients with osteoarthritis at the time of joint replacement surgery (median age 74, range 59-84), or from control subjects at autopsy (median age 74, range 50-81). Synovial tissue was fixed and processed into paraffin wax as described previously (Buckley et al. (1998), *ibid*)*.* In order to examine potential cross-reactivity of the carboxypeptidase-specific antibody with pancreatic carboxypeptidase, tissue sections were cut from macroscopically normal pancreas removed from two patients with pancreatic carcinoma. Six micrometer tissue sections were stained with antibody to carboxypeptidase. In double labelling experiments, alkaline phosphatase-conjugated tryptase-specific antibody was applied simultaneously with biotinylated monoclonal antibodies to carboxypeptidase. Extravidin^{®}-peroxidase conjugate (Sigma) was applied and immunostaining was developed using Fast Red TR and 3, 3'-diaminobenzidine. In further double staining studies, the carboxypeptidase-specific antibody, conjugated to alkaline phosphatase, was added with chymase-specific antibody CC1 that was biotinylated. Coded sections were analysed with the observer unaware of the tissue donor's disease status. A microscope with a *camera lucida* attachment (Leica, Milton Keynes) was used to co-localise protease staining in sequential sections of bronchus.

### Results

### Purification of carboxypeptidase

A milligram of purified carboxypeptidase was extracted from approximately 300 g skin tissue. The specific carboxypeptidase activity was 2.8 U/mg (2.8 µmol Hippuryl-DL-Arg substrate cleaved per minute per mg at 20 °C). Silver staining of the skin carboxypeptidase preparation on SDS-PAGE revealed a single diffuse band at a molecular weight of 33 kDa.

The sequence of the recombinant carboxypeptidase gene produced differed from the published sequence (Reynolds et al. (1992), Cloning and characterization of the novel gene for mast cell carboxypeptidase A, J. Clin. Invest. 89, 273-282) by two nucleotides (T90C and A96G), but since these discrepancies would have no effect at the amino acid level, it was not necessary to alter these. IPTG concentrations were optimised to increase the expression of carboxypeptidase in the soluble fraction of bacterial lysates. Western blotting of the lysates with the new antibodies prepared against skin carboxypeptidase revealed a unique band at a molecular weight of 36 kDa, of the expected size of the recombinant protein with the fusion partner.

### Characterisation of antibody specificity

Five hybridoma clones were isolated as sources of antibody to human skin carboxypeptidase and designated CA1, CA2, CA3, CA4 and CA5. Each of these clones produced antibodies of the IgG₁ subclass. The monoclonal antibodies reacted in ELISA to carboxypeptidase purified from skin as well as to the recombinant preparation of this protease, and this was confirmed in blotting studies. All of the antibodies reacted with cells with the characteristic morphology of mast cells in paraffin embedded lung, tonsil, pancreatic and synovial tissues and in GMA resin embedded bronchial biopsy tissues. There was no evidence for the staining of cells or structures other than mast cells. In the pancreas, there was staining of cells with the morphological appearance of mast cells, but not acinar cells, indicating the absence of cross-reactivity with pancreatic carboxypeptidase.

### Example 2: Mast cell carboxypetidase as marker for anaphylaxis and mastocytosis

### Methods

### Subjects

Serum samples from 183 separate cases of suspected anaphylaxis had been referred from hospitals throughout the UK (obtained through Dr Richard Pumphrey and Mr Colin Summers, Manchester). All samples had been collected within 8 h of the onset of the reaction. Serum samples were obtained from Dr Luis Escribano (Madrid) from patients with different categories of mastocytosis. Paediatric mastocytosis cases included those with urticaria pigmentosa (19 samples) and diffuse cutaneous mastocytosis (5). Adult cases comprised: pure cutaneous mastocytosis with only skin involvement (3), indolent systemic mastocytosis (31), systemic mastocytosis associated with recurrent anaphylaxis without skin lesions (6), well-differentiated systemic mastocytosis (4), aggressive systemic mastocytosis (7), mast cell leukaemia (1), and systemic mastocytosis associated with haematological non-mast-cell disease (7). As control groups, serum was collected from healthy blood donors attending the National Blood Transfusion Service, Southampton (more than 200 consecutively collected cases) from subjects with bronchial asthma (15) at Southampton General Hospital, and from children without a history of allergic disease (23). The Southampton and South West Hampshire Local Research Ethics Committees gave approval for these studies on an unlinked, anonymised basis.

### Purification of carboxypeptidase

Mast cell carboxypeptidase was extracted and purified from macroscopically normal human skin tissue obtained from amputated limbs or at apronectomy operations. Carboxypeptidase-like activity in extracts of skin in a high salt buffer (2M NaCl, 10mM MES, pH 6.5) was purified using an adaption of the method of (Goldstein et al. (1989), *ibid)* by affinity chromatography with potato tuber carboxypeptidase inhibitor coupled to agarose, followed by S-200 Sephacryl gel filtration chromatography (all reagents from Sigma, Poole, Dorset). The purified material was found to possess carboxypeptidase activity as monitored by cleavage of 1.5M hippuryl-L-Phe (Sigma) at 260nm. On SDS polyacrylamide gel electrophoresis, there was a major diffuse band of 34 kDa, which reacted with carboxypeptidase-specific monoclonal antibody CA1 on Western blotting.

### Development of ELISA for carboxypeptidase

Various combinations of monoclonal antibodies CA1, CA2, CA3, CA4 and CA5 were investigated in a sandwich ELISA procedure. Immunoglobulins were purified from hybridoma culture supernatants by ammonium sulphate precipitation (Buckley et al. (2001), Elevated serum concentrations of beta-tryptase, but not alpha-tryptase, in sudden infant death syndrome (SIDS). An investigation of anaphylactic mechanisms, Clin. Exp. Allergy 31, 1696-1704) followed by protein G affinity chromatography (Sigma). For use as detecting antibodies immunoglobulins were biotinylated.

Purified immunoglobulins were diluted in 50mM sodium carbonate (pH 9.6) coated overnight (4 °C) on microtitre plates (Nunc Maxisorp, Life Technologies, Paisley) in a humidified chamber. All subsequent steps were carried out at 22 °C (all in a total volume of 100 µl). After four washes with PBS containing 0.1% Tween 20 (PBS-T), non-specific binding sites were blocked with 3% bovine serum albumin (BSA) for 1 h, and after a further washing stage the standard carboxypeptidase preparation or sample were added to wells and incubated for 90 min. Biotinylated detecting antibody in culture supernatant diluted in PBS-T with 0.1% FCS was added following washing for a further 90 min, and after another washing stage, Extravidtin^{®}-horseradish peroxidise conjugate (Sigma) for 30 min. Plates were washed and a colour reaction in wells developed with 0.3 mg/ml ortho-phenylene diamine (OPD) in citrate phosphate buffer, pH 5.5 containing 0.01% H₂O₂. The reaction was stopped with 3M H₂SO₄, and the plates read at 490nm.

The method adopted as providing the most sensitive measurement, involved addition of monoclonal antibody CA4 as the capture antibody, and biotinylated antibody CA5 as detecting antibody. Carboxypeptidase purified from human skin was added at 0.25 - 64 ng/ml, diluted in PBS-T for the construction of a standard curve. Serum samples were added neat.

### ELISA validation

The assay was validated for use with serum and for mast cell supernatants, investigating the parallelism of dilution curves and the recovery of carboxypeptidase spiked into samples. The stability of purified carboxypeptidase or endogenous carboxypeptidase in pooled serum was investigated following incubation for various periods at 22 °C, and the effects of freeze-thawing examined.

### Results

Carboxypeptidase levels in serum or plasma collected within 8 hr of the onset of an allergic reaction were significantly greater than in those of either of the control groups (p<0.0001). In some cases the concentrations were more than 100-fold greater. In 83% of anaphylaxis cases with an elevated tryptase concentration there were carboxypeptidase levels greater than the normal range, though concentrations of these mast cell proteases were not correlated with each other. Out of 110 cases of suspected anaphylaxis that were tryptase-negative, there was an elevated concentration of carboxypeptidase in 77 (70%).

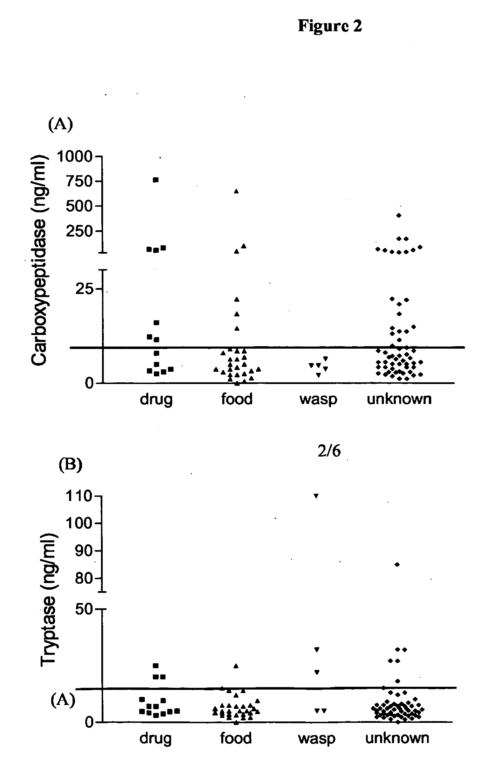

Serum levels of carboxypeptidase were greater than in healthy control groups for patients with various categories of disease associated with a high mast cell burden. As with the patients with anaphylaxis, high levels of carboxypeptidase were seen in cases of mast cell disease in which there was not a high circulating level of tryptase.
Additional data presented in Figure 2 provides further evidence that serum mast cell carboxpeptidase is a useful marker for anaphylaxis with increased levels in cases for which tryptase is not helpful. Figure 2 shows levels of both serum mast cell carboxypeptidase and tryptase in serum from cases where the allergen provoking the reaction was identified (drugs, food, wasp sting) or the trigger could not be identified despite various investigations (sometimes referred to as idiopathic anaphylaxis). This data again shows the value of measurement of serum mast cell carboxypeptidase in relation to anaphylaxis which may be missed by tryptase measurement, especially for example food-induced anaphylaxis which is generally accepted often results in no detectable increase in serum tryptase.

### Example 3: Detection of mast cell carboxpeptidase in saliva from individuals following a food-induced allergic reaction

### Methods

### Subjects

Three subjects were recruited who had a history of an allergic reaction to kiwi fruit, and three who had not experienced allergic symptoms on eating kiwi fruit.

### Food challenge

Saliva was collected from subjects prior to kiwi fruit being placed into the mouth. After 15 mins the fruit was spat out, and the mouth rinsed. Saliva was collected at regular intervals thereafter.

### Results

All of the allergic subjects experienced itching of the oral mucosa after the kiwi fruit was placed in the mouth, and in one case oedema was observed. Increased levels of carboxypeptidase were detected in the saliva of all allergic subjects from the first time point examined, and high levels were maintained for at least 60 min afterwards. Levels in control subjects were substantially lower than in the allergic subjects.

## Claims

1. Use of an immunoassay for mast cell carboxypeptidase to detect said carboxypeptidase in a sample derived from an individual following suspected anaphylaxis, or having suspected mastocytosis, wherein said use is for diagnosis of anaphylaxis or mastocytosis indicated by determination of an elevated level of said carboxypeptidase and said sample is a tryptase-negative serum, plasma or saliva sample.

2. A use according to claim 1 wherein said sample is a serum or plasma sample.

3. A use according to claim 2 wherein said sample is derived from a blood sample which was taken from an individual up to 24 hours from the onset of suspected anaphylaxis.

4. A use according to claim 3 wherein said sample is derived from a blood sample which was taken from an individual within 8 to 24 hours of the onset of suspected anaphylaxis

5. A use according to claim 4 wherein said sample is derived from a blood sample which was taken from an individual between 15 minutes to 3 hours of the onset of suspected anaphylaxis.

6. A use according to claim 1 wherein said sample is a saliva sample.

7. A use according to any one of claims 1 to 6 wherein said sample was taken from an individual following suspected anaphylaxis arising from ingestion of a food allergen or allergic reaction to a medication, or anaesthetic, or to an insect venom.

8. A use according to claim 7 wherein a saliva sample is employed which was collected between 15 minutes and 1 hour of ingestion of a suspected allergen.

9. A use according to claim1 or claim 2 employed for the diagnosis of categories of mastocytosis selected from diffuse cutaneous mastocytosis (DCM), urticaria pigmentosa (UP), mast cell leukaemia (MCL), aggressive systemic mastocytosis (ASM), indolent systemic mastocytosis (ISM) and mastocytosis without skin involvement associated to recurrent anaphylaxis or vascular collapse (SM-ANA).

10. A use according to any one of the preceding claims wherein assay for mast cell carboxypeptidase is carried out after negative testing for tryptase by immunoassay in the same sample or an identical sample.

11. A method of diagnosing an ingested allergen induced allergic reaction in an individual suspected of having suffered such a reaction which comprises determining by immunoassay mast cell carboxypeptidase present in a saliva sample from the individual wherein determination of an elevated level of said carboxypeptidase in the sample is indicative of an allergic reaction.

12. A method as claimed in claim 11 wherein said allergic reaction is food-induced anaphylaxis.

13. A method as claimed in claim 11 or claim 12 wherein said saliva sample was taken within 15 minutes to 1 hour of ingestion of the suspected allergen.

## Patentansprüche

1. Verwendung eines Immunoassays für Mastzellen-Carboxypeptidase zum Detektieren der Carboxypeptidase in einer Probe, erhalten von einem Individuum nach mutmaßlicher Anaphylaxie, oder der mutmaßliche Mastozytose aufweist, wobei die Verwendung für Diagnose von Anaphylaxie oder Mastozytose ist, angezeigt durch Bestimmung eines erhöhten Niveaus der Carboxypeptidase, und wobei die Probe eine Tryptase-negative Serum-, Plasma- oder Speichelprobe ist.

2. Verwendung nach Anspruch 1, wobei die Probe eine Serum- oder Plasmaprobe ist.

3. Verwendung nach Anspruch 2, wobei die Probe von einer Blutprobe erhalten wird, die von einem Individuum bis zu 24 Stunden vom Ausbruch der mutmaßlichen Anaphylaxie genommen wurde.

4. Verwendung nach Anspruch 3, wobei die Probe von einer Blutprobe erhalten wird, die von einem Individuum innerhalb von 8 bis 24 Stunden nach dem Ausbruch der mutmaßlichen Anaphylaxie genommen wurde.

5. Verwendung nach Anspruch 4, wobei die Probe von einer Blutprobe erhalten wird, die von einem Individuum innerhalb zwischen 15 Minuten bis 3 Stunden nach dem Ausbruch der mutmaßlichen Anaphylaxie genommen wurde.

6. Verwendung nach Anspruch 1, wobei die Probe eine Speichelprobe ist.

7. Verwendung nach einem der Ansprüche 1 bis 6, wobei die Probe von einem Individuum nach mutmaßlicher Anaphylaxie genommen wurde, entstehend aus Aufnahme eines Nahrungsmittel-Allergens oder einer allergischen Reaktion auf ein Medikament oder Anästhetikum oder auf ein Insektengift.

8. Verwendung nach Anspruch 7, wobei eine Speichelprobe eingesetzt wird, die zwischen 15 Minuten und 1 Stunde nach Aufnahme eines mutmaßlichen Allergens genommen wurde.

9. Verwendung nach Anspruch 1 oder Anspruch 2, eingesetzt für die Diagnose von Mastozytose-Kategorien, ausgewählt aus diffuser Haut-Mastozytose (DCM), Urticaria pigmentosa (UP), Mastzellen-Leukämie (MCL), aggressiver systemischer Mastozytose (ASM), indolenter systemischer Mastozytose (ISM) und Mastozytose ohne Hautbeteiligung, assoziiert mit rezidivierender Anaphylaxie oder vaskulärem Kollaps (SM-ANA).

10. Verwendung nach einem der vorstehenden Ansprüche, wobei ein Assay für Mastzellen-Carboxypeptidase nach negativem Testen auf Tryptase durch Immunoassay in derselben Probe oder einer identischen Probe ausgeführt wird.

11. Verfahren zum Diagnostizieren einer durch ein aufgenommenes Allergen induzierten allergischen Reaktion in einem Individuum, der mutmaßlich eine derartige Reaktion erlitten hat, das umfasst, durch Immunoassay Mastzellen-Carboxypeptidase zu bestimmen, die in einer Speichelprobe von dem Individuum vorhanden ist, wobei Bestimmung eines erhöhten Niveaus der Carboxypeptidase in der Probe eine allergische Reaktion anzeigt.

12. Verfahren nach Anspruch 11, wobei die allergische Reaktion Nahrungsmittelinduzierte Anaphylaxie ist.

13. Verfahren nach Anspruch 11 oder Anspruch 12, wobei die Speichelprobe innerhalb von 15 Minuten bis 1 Stunde nach Aufnahme des mutmaßlichen Allergens genommen wurde.

## Revendications

1. Utilisation d'un dosage immunologique sur une carboxypeptidase mastocytaire pour détecter ladite carboxypeptidase dans un échantillon dérivé d'un individu à la suite d'une anaphylaxie suspectée, ou ayant une mastocytose suspectée, où ladite utilisation est pour le diagnostic d'une anaphylaxie ou d'une mastocytose indiquée par la détermination d'un niveau élevé de ladite carboxypeptidase et ledit échantillon est un échantillon de sérum, de plasma ou de salive négatif à la tryptase.

2. Utilisation selon la revendication 1, dans laquelle ledit échantillon est un échantillon de sérum ou de plasma.

3. Utilisation selon la revendication 2, dans laquelle ledit échantillon est dérivé d'un échantillon de sang qui a été prélevé sur un individu jusqu'à 24 heures à partir du déclenchement d'une anaphylaxie suspectée.

4. Utilisation selon la revendication 3, dans laquelle ledit échantillon est dérivé d'un échantillon de sang qui a été prélevé sur un individu dans les 8 à 24 heures du déclenchement d'une anaphylaxie suspectée.

5. Utilisation selon la revendication 4, dans laquelle ledit échantillon est dérivé d'un échantillon de sang qui a été prélevé sur un individu entre 15 minutes et 3 heures du déclenchement d'une anaphylaxie suspectée.

6. Utilisation selon la revendication 1, dans laquelle ledit échantillon est un échantillon de salive.

7. Utilisation selon l'une quelconque des revendications 1 à 6, dans laquelle ledit échantillon a été prélevé sur un individu à la suite d'une anaphylaxie suspectée provenant d'une ingestion d'un allergène alimentaire ou d'une réaction allergique à une médication, ou un anesthésique, ou à un venin d'insecte.

8. Utilisation selon la revendication 7, dans laquelle un échantillon de salive est employé qui a été collecté entre 15 minutes et 1 heure d'ingestion d'un allergène suspecté.

9. Utilisation selon la revendication 1 ou la revendication 2 employée pour le diagnostic de catégories de mastocytoses choisies parmi une mastocytose cutanée diffuse (DCM), un urticaire pigmentaire (UP), une leucémie à mastocytes (MCL), une mastocytose systémique agressive (ASM), une mastocytose systémique indolente (ISM) et une mastocytose sans implication de la peau associée à une anaphylaxie récurrente ou un collapsus vasculaire (SM-ANA).

10. Utilisation selon l'une quelconque des revendications précédentes, dans laquelle le dosage sur une carboxypeptidase mastocytaire est réalisé après un test négatif pour la tryptase par un dosage immunologique dans le même échantillon ou un échantillon identique.

11. Méthode pour le diagnostic d'une réaction allergique induite par un allergène ingéré chez un individu suspecté d'avoir souffert d'une telle réaction qui comprend la détermination par un dosage immunologique d'une carboxypeptidase mastocytaire présente dans un échantillon de salive issu de l'individu, dans laquelle la détermination d'un niveau élevé de ladite carboxypeptidase dans l'échantillon est une indication d'une réaction allergique.

12. Méthode selon la revendication 11, dans laquelle ladite réaction allergique est une anaphylaxie induite par un aliment.

13. Méthode selon la revendication 11 ou la revendication 12, dans laquelle ledit échantillon de salive a été prélevé dans les 15 minutes à 1 heure d'ingestion de l'allergène suspecté.


### SEQUENCE LISTING

<110> University of Southampton WALLS, Andrew Finlay ZHOU, Xiaoying
<120> Mast Cell Carboxypeptidase As A marker For Anaphylaxis And Mastocytosis
<130> CI.P53726WO
<150> GB 0601058.1
   <151> 2006-01-19
<160> 2
<170> PatentIn version 3.3
<210> 1
   <211> 33
   <212> DNA
   <213> artificial
<220>
   <223> PCT Primer
<400> 1
   tttgatgtta aagaattcat cccaggcagg cac 33
<210> 2
   <211> 33
   <212> DNA
   <213> artificial
<220>
   <223> PCR Primer
<400> 2
   ttccaaacag aggaagcttc tataggaagt atg 33